(19) Europäisches Patentamt
European Patent Office
Office européen des brevets

(11) Veröffentlichungsnummer: 0 453 841 A2

# (12) EUROPÄISCHE PATENTANMELDUNG

(21) Anmeldenummer: 91105451.8

(22) Anmeldetag: 06.04.91

(51) Int. Cl.5: C07C 69/734, C07C 67/343, C07F 9/40

(30) Priorität: 21.04.90 DE 4012792

(43) Veröffentlichungstag der Anmeldung: 30.10.91 Patentblatt 91/44

(84) Benannte Vertragsstaaten: BE CH DE FR GB IT LI NL

(71) Anmelder: BAYER AG
W-5090 Leverkusen 1 Bayerwerk(DE)

(72) Erfinder: Gayer, Herbert, Dr.
Alfred-Delp-Strasse 4
W-4019 Monheim-Baumberg(DE)
Erfinder: Gerdes, Peter, Dr.
Augustastrasse 53
W-5100 Aachen(DE)
Erfinder: Klausener, Alexander, Dr.
Dahlerdyk 173
W-4150 Krefeld 1(DE)

## (54) Verfahren zur Herstellung von 1-Alkoxyhexatrien-2-carbonsäureestern.

(57) Beschrieben wird ein neues Verfahren zur Herstellung von 1-Alkoxyhexatrien-2-carbonsäureestern, welche als Antibiotica und Schädlingsbekämpfungsmittel bekannt sind, duroh Umsetzung von Oxophosphonsäureestern mit Alkoxycarbonylalkylphosphonester-Derivaten und anschließender Umsetzung der so erhaltenen Zwischenprodukte mit Ameisensäuremethylester und einem Methylierungsmittel zu Alkoxymethylencarbonester-phosphonsäureestern und letztendlicher Umsetzung dieser neuen Zwischenprodukte mit Ketonen oder Aldehyden zu den Zielverbindungen.

EP 0 453 841 A2

Die Erfindung betrifft ein neues Verfahren zur Herstellung von 1-Alkoxyhexatrien-2-carbonsäureestern, welche als Antibiotica und Schädlingsbekämpfungsmittel bekannt sind, sowie neue Zwischenprodukte zur Herstellung derselben.

Es ist bekannt, daß man bestimmte substituierte 1-Alkoxyhexatrien-2-carbonsäureester erhält, wenn man substituierte Pentadiencarbonsäureester in einer vielstufigen Synthese zunächst mit Lithiumaluminiumhydrid reduziert, anschließend mit Mangandioxid oxidiert, danach mit Lithiumorganischen Verbindungen in Gegenwart von Quecksilbersalzen umsetzt, anschließend ein weiteres Mal mit Mangandioxid oxidiert und schließlich eine Wittig-Reaktion mit Methoxymethylentriphenylphosphoran anschließt (vergleiche Tetrahedron Lett. 28, 475 - 478 [1987]). Nachteilig bei diesem Verfahren und prohibitiv für eine technische Durchführbarkeit sind einerseits die Reagenzien Lithiumaluminiumhydrid, die Quecksilbersalze und die Lithiumorganischen Verbindungen sowie auch das in der letzten Stufe verwendete Methoxymethylentriphenylphosphoran, welches aus dem extrem toxischen Methoxymethylenchlorid hergestellt wird, andererseits auch die Vielzahl der Reaktionsstufen, die eine geringe Ausbeute bedingt und aufwendige Reinigungsschritte erforderlich macht. Nicht zuletzt ist auch die erforderliche Reaktionstemperatur von -70 °C ein schwerwiegendes Hindernis für großtechnische Synthesen.

Weiterhin ist eine dreistufige Synthese bekannt, die ebenfalls zu substituierten 1-Alkoxyhexatrien-2-carbonsäureestern führt, wobei zunächst Zimtaldehyd mit 2-Oxobuttersäure kondensiert wird, anschließend die Säurefunktion mit Thionylchlorid in Methanol verestert wird und der so erhältliche 2-Oxocarbonester in der letzten Stufe wiederum mit Methoxymethylentriphenylphosphoran in einer Wittig-Reaktion umgesetzt wird (vergleiche DE-OS 30 25 368; Liebigs Ann. Chem. 1984, 1616 - 1625). Auch bei diesem Verfahren muß das Methoxymethylentriphenylphosphoran vorher aus dem hochtoxischen Methoxymethylenchlorid hergestellt werden.

Nachteilig bei allen derartigen Wittig-Reaktionen sind außerdem die Probleme die durch freiwerdendes Triphenylphosphinoxid entstehen, eine Verbindung, die sich aus allen Reaktionsgemischen nur sehr schwierig und mit aufwendigen Reinigungsoperationen abtrennen läßt.

Es wurde nun gefunden, daß man 1-Alkoxyhexatrien-2-carbonsäureester der allgemeinen Formel (I),

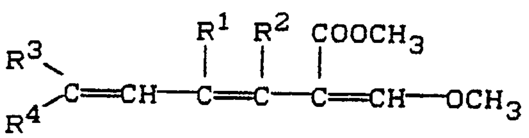

(I)

in welcher

$R^1$ für Wasserstoff, für gegebenenfalls substituiertes Alkyl, für Alkenyl, Alkinyl, für Cycloalkyl, Cycloalkenyl, für Phenyl oder für Heterocyclyl steht oder für einen Rest der Formel $-O-R^5$; $-S-R^5$ oder

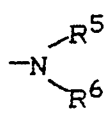

steht,

$R^2$ für Wasserstoff, für gegebenenfalls substituiertes Alkyl, für Alkenyl, Alkinyl, für Cycloalkyl, Cycloalkenyl, für Phenyl oder für Heterocyclyl steht oder

$R^1$ und $R^2$ gemeinsam mit den Kohlenstoffatomen, an welche sie gebunden sind, für einen jeweils gegebenenfalls substituierten Carbo- oder Heterocyclus stehen und

$R^3$, $R^4$, $R^5$ und $R^6$ unabhängig voneinander jeweils für Wasserstoff, für gegebenenfalls substituiertes Alkyl, für Alkenyl, Alkinyl, für Cycloalkyl, Cycloalkenyl, für gegebenenfalls substituiertes Phenyl oder für gegebenenfalls substituiertes Heterocyclyl stehen, wobei

$R^3$ und $R^4$ auch gemeinsam für einen zweifach verknüpften Alkandiylrest stehen können,

erhält, wenn man Oxophosphonsäureester der Formel (II),

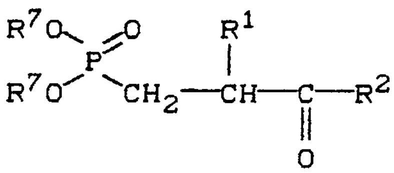

(II)

in welcher

$R^1$ und $R^2$ die oben angegebene Bedeutung haben und

$R^7$ für Wasserstoff, für gegebenenfalls substituiertes Alkyl, für Alkenyl, Alkinyl, für Cycloalkyl, Cycloalkenyl, für Phenyl oder für Heterocyclyl steht,

zunächst in einer ersten Stufe mit Alkoxycarbonylalkylphosphonester-Derivaten der Formel (III),

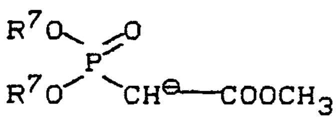

$M^{\oplus}$ (III)

in welcher

$R^7$ die oben angegebene Bedeutung hat und

$M^{\oplus}$ für ein Alkalimetallkation steht,

in Gegenwart eines Verdünnungsmittels und gegebenenfalls in Gegenwart einer Base umsetzt, anschließend in einer zweiten Stufe die so erhältlichen Acrylesterderivate der Formel (IV),

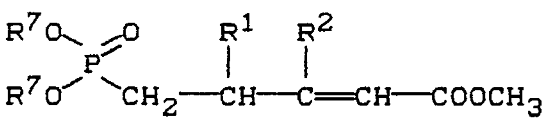

(IV)

in welcher

$R^1$, $R^2$ und $R^7$ die oben angegebene Bedeutung haben,

mit Ameisensäuremethylester in Gegenwart eines Verdünnungsmittels und in Gegenwart einer Base formyliert, dann entweder direkt in Anschluß daran in einem Reaktionsschritt oder auch in einer getrennten Reaktionsstufe mit einem Methylierungsmittel gegebenenfalls in Gegenwart eines Verdünnungsmittels und gegebenenfalls in Gegenwart einer Base methyliert und in einer letzten Stufe die so erhältlichen Alkoxymethylencarbonester der Formel (V),

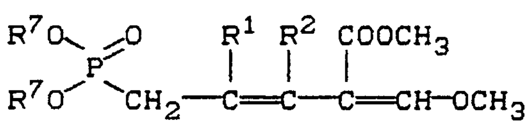

(V)

in welcher

$R^1$, $R^2$ und $R^7$ die oben angegebene Bedeutung haben,

mit Ketonen oder Aldehyden der Formel (VI),

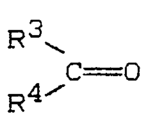

(VI)

in welcher

$R^3$ und $R^4$ die oben angegebene Bedeutung haben,

gegebenenfalls in Gegenwart eines Verdünnungsmittels und in Gegenwart einer Base umsetzt.

Dabei ist es als ausgesprochen überraschend anzusehen, daß die Formylierung der Acrylesterderivate der Formel (IV) mit Ameisensäureester in Gegenwart einer starken Base mit sehr hoher Selektivität an der Doppelbindung in Nachbarstellung zur Estergruppe erfolgt, da aus der Literatur bekannt ist, daß die Formylierung der ähnlichen Systeme Cyclopentylidenessigsäureethylester und Crotonsäureethylester in Allylstellung zur Doppelbindung erfolgt, wobei die Verbindungen [2-Formylcyclopentyliden]-essigsäureethylester (vergleiche J. Univ. Bombay 12, Tl. 3A, 66, [1943] bzw. Beilstein Bd. 10, E3, 2900 - 2901) und 4-Formylcrotonsäureethylester (vergleiche Liebigs Ann. Chem. 505, 193 [1933]) gebildet werden. Im erfindungsgemäßen Verfahren findet die Formylierung in Allylstellung überhaupt nicht und eine alternative Formylierung in Nachbarstellung zum Phosphor, welche durch eine eventuelle Verschiebung der Doppelbindung in $\beta$, $\gamma$-Position zur Carbonestergruppe begünstigt würde, oft nur in einer Nebenreaktion mit einem Anteil an der Gesamtumsetzung von weniger als 10 % statt, die Nebenprodukte sind darüber hinaus in der nächsten Reaktionsstufe völlig inert und lassen sich daher leicht aus dem Reaktionsgemisch abtrennen.

Das erfindungsgemäße Verfahren besitzt gegenüber dem Stand der Technik den Vorteil, daß die erwünschten Endprodukte in deutlich besserer Ausbeute mit leicht zugänglichen und auch im großtechnischen Maßstab gut handhabbaren Ausgangsmaterialien erhältlich sind und aufwendige Reinigungsoperationen entfallen, da beispielsweise freiwerdende Phosphorsäureestersalze durch wässrige Aufarbeitung wesentlich leichter abzutrennen sind als das bei den bisher angewandten Verfahren als Nebenprodukt anfallende Triphenylphosphinoxid.

Die mit Hilfe des erfindungsgemäßen Verfahrens erhältlichen 1-Alkoxyhexatrien-2-carbonsäureester sind durch die Formel (I) allgemein definiert.

Bevorzugt herstellbar sind Verbindungen der Formel (I), bei welchen

$R^1$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen, Cyano, Nitro, jeweils geradkettiges oder verzweigtes Alkenyl, Alkinyl, Alkoxy, Alkenyloxy, Alkinyloxy, Alkylthio, Alkylendioxy, Alkoxycarbonyl oder Alkoximino mit jeweils 1 bis 6 Kohlenstoffatomen, jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, Cycloalkyl oder Cycloalkenyl mit jeweils 3 bis 7 Kohlenstoffatomen oder jeweils gegebenenfalls ein- bis fünffach gleich oder verschieden substituiertes Phenyl, Phenoxy, Phenylthio, Benzyloxy oder Benzylthio, wobei als Phenylsubstituenten in den letztgenannten Fällen jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^1$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht; und außerdem für einen Rest der Formel -O-$R^5$; -S-$R^5$ oder

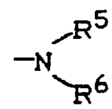

steht,

$R^2$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^1$ genannten Alkylsubstituenten infrage kommen;
außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^1$ und $R^2$ gemeinsam mit den Kohlenstoffatomen, an welche sie gebunden sind, für einen jeweils gegebenenfalls ein- bis fünffach, gleich oder verschieden substituierten, fünf- bis siebengliedrigen Carbo- oder Heterocyclus mit 1 bis 3 Heteroatomen stehen, wobei als Heteroatome Stickstoff, Sauerstoff oder Schwefel infrage kommen und wobei als Substituenten jeweils infrage kommen: Halogen sowie jeweils geradkettiges oder verzweigtes Alkyl, Alkoxy oder Alkylthio mit jeweils 1 bis 4 Kohlenstoffatomen,

$R^3$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^1$ genannten Alkylsubstituenten infrage kommen;

außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen oder für jeweils gegebenenfalls einfach oder mehrfach gleich oder verschieden substituiertes Phenyl oder Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, und wobei als Substituenten jeweils infrage kommen: Halogen, jeweils geradkettiges oder verzweigtes Alkyl, Alkenyl, Alkinyl, Alkoxy, Alkenyloxy oder Alkinyloxy mit jeweils bis zu 4 Kohlenstofftomen, sowie jeweils gegebenenfalls ein- bis fünffach, gleich oder verschieden durch Halogen sowie jeweils geradkettiges oder verzweigtes Alkyl oder Alkoxy substituiertes Phenyl, Phenoxy oder Benzyloxy und

$R^4$, $R^5$ und $R^6$ unabhängig voneinander für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^1$ genannten Alkylsubstituenten infrage kommen;

außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^3$ und $R^4$ gemeinsam für einen zweifach verknüpften Alkandiylrest mit 3 bis 6 Kohlenstoffatomen stehen.

Besonders bevorzugt herstellbar sind Verbindungen der Formel (I), bei welchen

$R^1$ für Wasserstoff, Methyl, Ethyl, n- oder i-Propyl, n-, i-, s- oder t-Butyl, Methoxy, Ethoxy, n- oder i-Propoxy, Methylthio, Allyl, Propargyl, für Cyclohexyl, Cyclohexenyl, Cyclopentyl, Cyclopentenyl, für Phenyl oder für jeweils gegebenenfalls ein- bis fünffach gleich oder verschieden im Phenylteil substituiertes Benzyl oder Phenoxy steht, wobei als Substituenten jeweils infrage kommen:

Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Difluormethoxy, Trifluormethylthio,

$R^2$ für Wasserstoff, Methyl, Ethyl, n- oder i-Propyl, n-, i-, s- oder t-Butyl, für Cyclopentyl, Cyclopentenyl, Cyclohexyl, Cyclohexenyl oder Phenyl steht oder

$R^1$ und $R^2$ gemeinsam mit den Kohlenstoffatomen, an welche sie gebunden sind, für einen jeweils gegebenenfalls ein- bis dreifach, gleich oder verschieden substituierten fünf- bis siebengliedrigen Carbo- oder Heterocyclus mit 1 bis 2 Heteroatomen stehen, wobei als Heteroatome Stickstoff, Sauerstoff oder Schwefel infrage kommen und wobei als Substituenten jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio,

$R^3$ für gegebenenfalls ein- bis dreifach gleich oder verschieden substituiertes Phenyl oder für gegebenenfalls ein- bis zweifach gleich oder verschieden substituiertes Heteroaryl steht, wobei Heteroaryl für einen aromatischen 5-Ring- oder 6-Ring-Heterocyclus mit 1 oder 2 gleichen oder verschiedenen Heteroatomen aus der Gruppe Stickstoff, Sauerstoff oder Schwefel steht und wobei als Substituenten jeweils infrage kommen:

Fluor, Chlor, Brom, Methyl, Ethyl, n- oder i-Propyl, n-, i-, s- oder t-Butyl, Allyl, Propargyl, Methoxy, Ethoxy, n- oder i-Propoxy, Allyloxy, Propargyloxy sowie jeweils gegebenenfalls

ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, substituiertes Phenyl, Phenoxy oder Benzyloxy,

$R^4$ für Wasserstoff, Methyl, Ethyl, n- oder i-Propyl, n-, i-, s- oder t-Butyl steht oder

$R^3$ und $R^4$ gemeinsam für einen 1,3-Propandiyl, 1,4-Butandiyl, 1,5-Pentandiyl oder 1,6-Hexandiylrest stehen.

Ganz besonders bevorzugt herstellbar sind Verbindungen der Formel (I), bei welchen

$R^1$ für Wasserstoff, Methyl, Methoxy, Phenyl oder Phenoxy steht,

$R^2$ für Wasserstoff oder Methyl steht oder

$R^1$ und $R^2$ gemeinsam mit den Kohlenstoffatomen, an welche sie gebunden sind, für einen 5-gliedrigen oder 6-gliedrigen Carbo- oder Heterocyclus mit einem Sauerstoff oder Schwefel als Heteroatom stehen, der gegebenenfalls ein- bis dreifach durch Methyl substituiert sein kann,

$R^3$ für gegebenenfalls einfach oder zweifach, gleich oder verschieden substituiertes Phenyl oder für gegebenenfalls einfach oder zweifach, gleich oder verschieden substituiertes Heteroaryl steht, wobei als Heteroarylreste die folgenden infrage kommen:

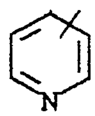 ; 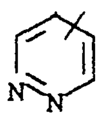 ; 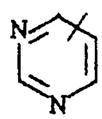 ; 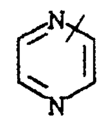 ;

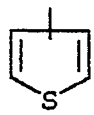 ; 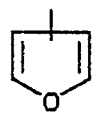 ; 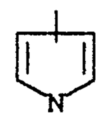 ; 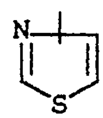 ;

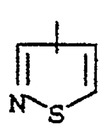 ; 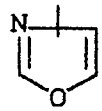 ; 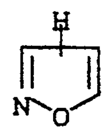

und wobei als Phenyl- oder Heteroarylsubstituenten jeweils infrage kommen: Fluor, Chlor, Brom, Methyl, Ethyl, n- oder i-Propyl, n-, i-, s- oder t-Butyl, Methoxy, Ethoxy, Allyloxy, Propargyloxy sowie jeweils gegebenenfalls ein- bis dreifach, gleich oder verschieden durch Fluor, Chlor, Brom, Methyl, Ethyl, Methoxy oder Ethoxy substituiertes Phenyl, Phenoxy oder Benzyloxy,

$R^4$ für Wasserstoff steht oder

$R^3$ und $R^4$ gemeinsam für einen 1,4-Butandiylrest oder einen 1,5-Pentandiylrest stehen.

Insbesondere bevorzugt herstellbar sind Verbindungen der Formel (I), bei welchen

$R^1$ für Wasserstoff und

$R^2$ für Methyl steht oder

$R^1$ und $R^2$ gemeinsam mit den Kohlenstoffatomen, an welche sie gebunden sind, für einen Rest der Formel

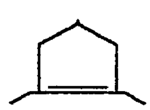 ; 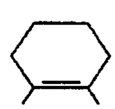 oder 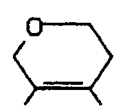

stehen,

$R^3$ für gegebenenfalls einfach oder zweifach, gleich oder verschieden substituiertes Phenyl steht, wobei als Substituenten infrage kommen:

Chlor, Phenyl, Phenoxy oder Benzyloxy und wobei als Substitutionsstelle im Phenylring die 3-Position besonders bevorzugt ist und

$R^4$ für Wasserstoff steht.

Verwendet man beispielsweise $\gamma$-Ketobutylphosphonsäurediethylester und Methoxycarbonylmethan-

phosphonsäuredimethylester Kaliumsalz als Ausgangsstoffe, so läßt sich der Reaktionsablauf des erfindungsgemäßen Verfahrens durch das folgende Formelschema darstellen:

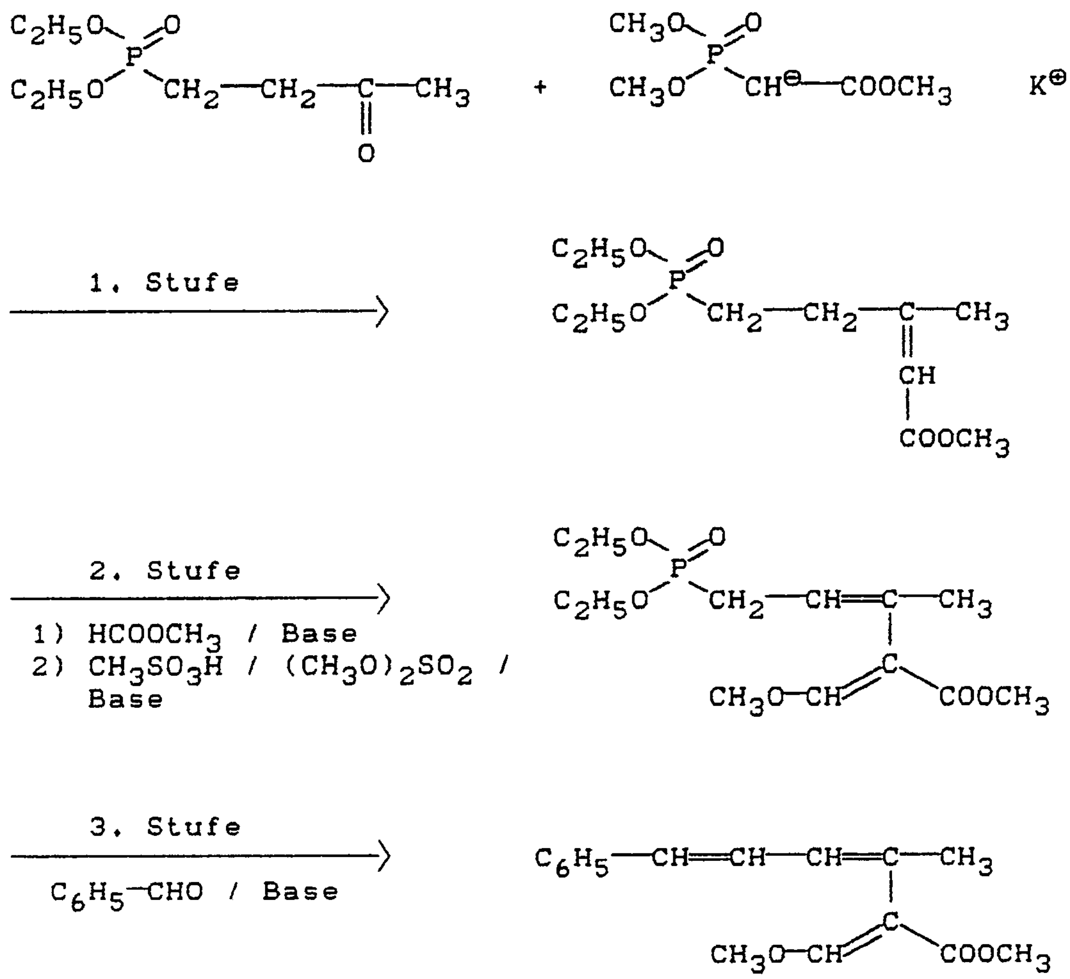


Die zur Durchführung des erfindungsgemäßen Verfahrens als Ausgangsstoffe verwendeten Oxophosphonsäureester sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäß herstellbaren Stoffe der Formel (I) als bevorzugt für diese Substituenten genannt wurden. $R^7$ steht vorzugsweise für Alkyl, Aralkyl oder Aryl, insbesondere für Methyl, Ethyl, n- oder i-Propyl, Benzyl oder Phenyl.

Die Oxophosphonsäureester der Formel (II) sind größtenteils bekannt oder erhältlich in Analogie zu bekannten Verfahren (vergleiche z. B. J. Amer. Chem. Soc. 77, 3101 - 3103 [1955]; CA 68, 220159; Tetrahedron 22, 2561 - 2573 [1966]; Tetrahedron 37, 1377 - 1384 [1981]; J. Med. Chem. 23, 300 - 304 [1980]).

Die zur Durchführung des erfindungsgemäßen Verfahrens weiterhin als Ausgangsstoffe benötigten Alkoxycarbonylalkylphosphonester-Derivate sind durch die Formel (III) allgemein definiert. In dieser Formel (III) steht $R^7$ vorzugsweise für diejenigen Reste, die bereits im Zusammenhang mit der Beschreibung der Vorprodukte der Formel (II) als bevorzugt für diesen Substituenten genannt wurden.

$M^{\oplus}$ steht vorzugsweise für ein Natrium- oder Kaliumkation.

Die Alkoxycarbonylalkylphosphonester der Formel (III) sind allgemein bekannt (vergleiche z. B. J. Org. Chem. 29, 3327 - 3330 [1964]; DE 21 62 571; Chem. Berichte 57, 1031 [1924]; CA 93: 79890s; CA 68: 220159).

Die zur Durchführung des erfindungsgemäßen Verfahrens weiterhin als Ausgangsstoffe benötigten Ketone oder Aldehyde sind durch die Formel (VI) allgemein definiert. In dieser Formel (VI) stehen $R^3$ und $R^4$ vorzugsweise für diejenigen Reste, die bereits im Zusammenhang mit der Beschreibung der erfindungsgemäß herstellbaren Stoffe der Formel (I) als bevorzugt für diese Substituenten genannt wurden.

Die Aldehyde und Ketone der Formel (VI) sind allgemein bekannte Verbindungen der organischen Chemie.

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether wie Diethylether, Dioxan, Tetrahydrofuran oder Ethylenglykoldimethyl- oder -diethylether, Amide wie Dimethylformamid, Dimethylacetamid, N-Methylformanilid, N-Methylpyrrolidon oder Hexamethylphosphorsäuretriamid, Sulfoxide wie Dimethylsulfoxid oder Alkohole wie Methanol oder Ethanol.

Die 1. Stufe des erfindungsgemäßen Verfahrens wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Kaliumhydrid, Natriumamid, Natriumhydroxid, Natriummethylat, Natriumethylat oder Kalium-t-butylat.

Die Reaktionstemperaturen können bei der Durchführung der ersten Stufe des erfindungsgemäßen Verfahrens in einem größeren Bereich variiert werden. Im allgemeinen arbeitet man bei Temperaturen von -20 °C bis 100 °C, vorzugsweise bei Temperaturen von 0 °C bis 30 °C.

Zur Durchführung der 1. Stufe des erfindungsgemäßen Verfahrens setzt man pro Mol an Oxophosphonsäureester der Formel (II) im allgemeinen 1,0 bis 1.5 Mol, vorzugsweise 1,0 bis 1.2 Mol an Alkoxycarbonylalkylphosphonester-Derivat der Formel (III) und gegebenenfalls 1.0 bis 3.0 Mol, vorzugsweise 1.0 bis 1.5 Mol an Base ein. Die Reaktionsdurchführung, Aufarbeitung und Isolierung der Reaktionsprodukte erfolgt nach allgemein üblichen Methoden (vergleiche z. B. Organic Reactions, Vol. 25, 73 [1977]).

In einer bevorzugten Ausführungsform geht man so vor, daß zunächst aus dem Alkoxycarbonylalkylphosphonester-Derivat der Formel (IIIa),

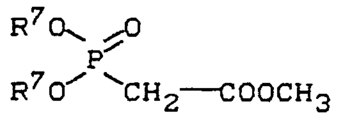

(IIIa)

in welcher

$R^7$ die oben angegebene Bedeutung hat,

mit äquivalenten Mengen einer geeigneten Base in einem geeigneten Lösungsmittel das entsprechende Salz der Formel (III) erzeugt wird. Zu dieser Lösung oder Suspension gibt man dann in kleinen Portionen den Oxophosphonester der Formel (II), rührt mehrere Stunden bei der erforderlichen Temperatur und arbeitet nach Standardverfahren auf.

Auf diese Art und Weise erhält man Acrylesterderivate der Formel (IV).

Diese Acrylesterderivate der Formel (IV) sind teilweise vorbeschrieben (vergleiche z. B. J. Chem. Res., Synop., 10, 378 - 379 [1986] bzw. CA 107: 7281d).

Noch nicht bekannt und ebenfalls Gegenstand der Erfindung sind Acrylesterderivate der Formel (IVa),

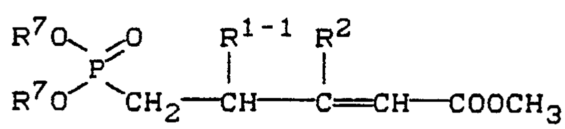

(IVa)

in welcher

$R^{1-1}$ für Wasserstoff, für gegebenenfalls substituiertes Alkyl, für Alkenyl, Alkinyl, für Cycloalkyl, Cycloalkenyl, für Phenyl oder für Heterocyclyl steht oder für einen Rest der Formel $-O-R^5$ oder $-S-R^5$ steht,

$R^2$ für Wasserstoff, für gegebenenfalls substituiertes Alkyl, für Alkenyl, Alkinyl, für Cycloalkyl, Cycloalkenyl, für Phenyl oder für Heterocyclyl steht oder

$R^{1-1}$ und $R^2$ gemeinsam mit den Kohlenstoffatomen, an welche sie gebunden sind, für einen jeweils gegebenenfalls substituierten Carbo- oder Heterocyclus stehen und

$R^5$ für Wasserstoff, für gegebenenfalls substituiertes Alkyl, für Alkenyl, Alkinyl, für Cycloalkyl, Cycloalkenyl, für gegebenenfalls substituiertes Phenyl oder für gegebenenfalls

substituiertes Heterocyclyl steht und

$R^7$ für Wasserstoff, für gegebenenfalls substituiertes Alkyl, für Alkenyl, Alkinyl, für Cyclkoalkyl, Cycloalkenyl, für Phenyl oder für Heterocyclyl steht.

Bevorzugt sind Verbindungen der Formel (IVa), bei welchen

$R^{1-1}$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen, Cyano, Nitro, jeweils geradkettiges oder verzweigtes Alkenyl, Alkinyl, Alkoxy, Alkenyloxy, Alkinyloxy, Alkylthio, Alkylendioxy, Alkoxycarbonyl oder Alkoximino mit jeweils 1 bis 6 Kohlenstoffatomen, jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, Cycloalkyl oder Cycloalkenyl mit jeweils 3 bis 7 Kohlenstoffatomen oder jeweils gegebenenfalls ein- bis fünffach gleich oder verschieden substituiertes Phenyl, Phenoxy, Phenylthio, Benzyloxy oder Benzylthio, wobei als Phenylsubstituenten in den letztgenannten Fällen jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^{1-1}$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht; und außerdem für einen Rest der Formel -O-$R^5$ oder -S-$R^5$ steht,

$R^2$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen;
außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^{1-1}$ und $R^2$ gemeinsam mit den Kohlenstoffatomen, an welche sie gebunden sind, für einen jeweils gegebenenfalls ein- bis fünffach, gleich oder verschieden substituierten, fünf- bis siebengliedrigen Carbo- oder Heterocyclus mit 1 bis 3 Heteroatomen stehen, wobei als Heteroatome Stickstoff, Sauerstoff oder Schwefel infrage kommen und wobei als Substituenten jeweils infrage kommen: Halogen sowie jeweils geradkettiges oder verzweigtes Alkyl, Alkoxy oder Alkylthio mit jeweils 1 bis 4 Kohlenstoffatomen und

$R^5$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen;
außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, und

$R^7$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen;

$R^7$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht.

Besonders bevorzugt sind Verbindungen der Formel (IVa), bei welchen

$R^{1-1}$ für Wasserstoff, Methyl, Ethyl, n- oder i-Propyl, n-, i-, s- oder t-Butyl, Methoxy, Ethoxy, n- oder i-Propoxy, Methylthio, Allyl, Propargyl, für Cyclohexyl, Cyclohexenyl, Cyclopentyl, Cyclopentenyl, für Phenyl oder für jeweils gegebenenfalls ein- bis fünffach gleich oder verschieden im Phenylteil substituiertes Benzyl oder Phenoxy steht, wobei als Substituenten jeweils infrage kommen:
Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Difluormethoxy, Trifluormethylthio,

$R^2$ für Wasserstoff, Methyl, Ethyl, n- oder i-Propyl, n-, i-, s- oder t-Butyl, für Cyclopentyl, Cyclopentenyl, Cyclohexyl, Cyclohexenyl oder Phenyl steht oder

$R^{1-1}$ und $R^2$ gemeinsam mit den Kohlenstoffatomen, an welche sie gebunden sind, für einen jeweils gegebenenfalls ein- bis dreifach, gleich oder verschieden substituierten fünf- bis siebengliedrigen Carbo- oder Heterocyclus mit 1 bis 2 Heteroatomen stehen, wobei als Heteroatome Stickstoff, Sauerstoff oder Schwefel infrage kommen und wobei als Substituenten jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio und

$R^7$ für Methyl, Ethyl, n- oder i-Propyl, n-, i-, s- oder t-Butyl, für Benzyl oder Phenyl steht.

Ganz besonders bevorzugt sind Verbindungen der Formel (IV), bei welchen

$R^{1-1}$ für Wasserstoff, Methyl, Methoxy, Phenyl oder Phenoxy steht,

$R^2$ für Wasserstoff oder Methyl steht oder

$R^{1-1}$ und $R^2$ gemeinsam mit den Kohlenstoffatomen, an welche sie gebunden sind, für einen 5-gliedrigen oder 6-gliedrigen Carbo- oder Heterocyclus mit einem Sauerstoff oder Schwefel als Heteroatom stehen, der gegebenenfalls ein- bis dreifach durch Methyl substituiert sein kann und

$R^7$ für Methyl, Ethyl, Phenyl oder Benzyl steht.

Insbesondere bevorzugt sind Verbindungen der Formel (IVa), bei welchen

$R^{1-1}$ für Wasserstoff und

$R^2$ für Methyl steht oder

$R^{1-1}$ und $R^2$ gemeinsam mit den Kohlenstoffatomen, an welche sie gebunden sind, für einen Rest der Formel

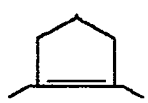 ; 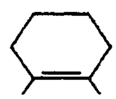 oder 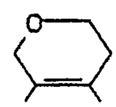

stehen und

$R^7$ für Methyl oder Ethyl steht.

Die Verbindungen der Formel (IVa) können in verschieden stereoisomeren Formen vorkommen; sowohl die reinen Isomeren als auch deren Gemische werden erfindungsgemäß beansprucht.

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether wie Diethylether, Dioxan, t-Butylmethylether, Tetrahydrofuran oder Ethylenglykoldimethyl- oder -diethylether, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die 2. Stufe des erfindungsgemäßen Verfahrens wird in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide oder -alkoholate, wie beispielsweise Natriumhydrid, Natriumamid, Natriumhydroxid, Natriummethylat, Natriumethylat oder, Kalium-t-butylat.

Die Reaktionstemperaturen können bei der Durchführung der 2. Stufe des erfindungsgemäßen Verfahrens in einem größeren Bereich variiert werden. Im allgemeinen arbeitet man bei Temperaturen von -20 °C bis 100 °C, vorzugsweise bei Temperaturen von 0 °C bis 60 °C.

Als Methylierungsmittel zur Durchführung der zweiten Stufe des erfindungsgemäßen Verfahrens kommen alle üblicherweise verwendbaren Methylierungsmittel wie beispielsweise Dimethylsulfat, p-Toluolsulfonsäuremethylester, Methyliodid, Methylbromid oder Methylchlorid infrage. Mit besonderem Vorzug verwendet man Dimethylsulfat.

Zur Durchführung der 2. Stufe des erfindungsgemäßen Verfahrens setzt man pro Mol an Acrylester der

Formel (IV) im allgemeinen 1.0 bis 20.0 Mol, vorzugsweise 1.0 bis 15.0 Mol an Ameisensäuremethylester, 1.0 bis 1.5 Mol, vorzugsweise 1.0 bis 1.2 Mol an Methylierungsmittel sowie 1.0 bis 10.0 Mol, vorzugsweise 1.0 bis 5.0 Mol an Base ein.

Man kann dabei entweder die Reaktion in zwei getrennten Stufen d. h. zuerst die Formylierung und dann die Alkylierung durchführen; dabei setzt man zunächst die Acrylester der Formel (IV) mit dem Ameisensäuremethylester in Gegenwart einer Base und in Gegenwart eines Verdünnungsmittels bei Temperaturen zwischen 0° C und 40° C um und isoliert dann die entsprechenden Enole (bzw. Enolate) der Formel (VII),

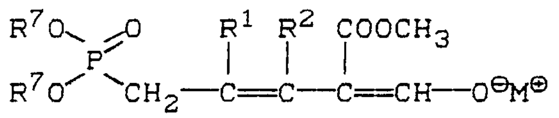

(VII)

in welcher

$R^1$, $R^2$ und $R^7$ die oben angegebene Bedeutung haben und

$M^\oplus$ für Wasserstoff oder das Äquivalent eines Alkali- oder Erdalkalikations (vorzugsweise für ein Natrium- oder Kaliumkation) steht,

in der Regel in Form ihrer Salze.

Aus den Salzen können die freien Enole durch Ansäuern mit verdünnten Säuren, wie beispielsweise verdünnte Salzsäure und darauffolgende Extraktion mit einem geeigneten organischen Lösungsmittel wie beispielsweise Essigester isoliert werden.

Die freien Enole können dann in einem geeigneten Verdünnungsmittel in Gegenwart einer geeigneten Base mit dem Methylierungsmittel bei Temperaturen von 20° C bis 60° C methyliert werden.

Alternativ ist es möglich, die Enolatsalze der Formel (VII) direkt in Reaktionsgemisch mit dem Methylierungsmittel umzusetzen. In einer besonders bevorzugten Variante dieser "Eintopfreaktion" arbeitet man zunächst im Verlauf der Formylierung mit 2 Äquivalenten Basen, wie beispielsweise Natriummethylat oder Natriumhydrid; man erhält dadurch in der Reaktionsmischung das Enolat der Formel (VII) in Form des Natriumsalzes und als Nebenprodukt aus dem Ameisensäuremethylester Natriummethylat. Dieses Nebenprodukt läßt sich mit einer geeigneten Säure wie beispielsweise Methansulfonsäure protonieren, darauf gibt man zusammen mit einem weiteren Äquivalent Base, wie beispielsweise Kaliumcarbonat, das Methylierungsmittel zu.

Durch diese Vorgehensweise, erübrigt es sich, das Methylierungsmittel im Überschuß einzusetzen, was sonst erforderlich wäre, um Verluste durch seine Reaktion mit Natriummethylat auszugleichen.

Für den Fall, daß $R^7$ verschieden von Methyl ist, tritt jedoch bei dieser Vorgehensweise eine teilweise Umesterung im Phosphonesterteil der Verbindungen (V) und (VII) auf, wobei gemischte Phosphonester als Nebenprodukte entstehen können. Da diese sich jedoch in der folgenden 3. Stufe des erfindungsgemäßen Verfahrens völlig analog verhalten wie die eingesetzten Phosphonestergruppen, ist es in der Regel nicht notwendig, sie aus dem Reaktionsprodukt abzutrennen.

Die so erhältlichen Alkoxymethylencarbonester der Formel (V) sind ebenso wie die Enole der Formel (VII) bzw. deren Enolatsalze noch nicht bekannt und ebenfalls Gegenstand der Erfindung. Für die Substituentendefinitionen $R^1$, $R^2$ und $R^7$ sowie deren Kombinationen gelten die gleichen Vorzugsbereiche wie weiter vorne für die Verbindungen der Formeln (I) und (IVa) angegeben.

Die Verbindungen der Formeln (V) und (VII) können in verschieden stereoisomeren Formen vorkommen; sowohl die reinen Isomeren als auch deren Gemische werden erfindungsgemäß beansprucht.

Als Verdünnungsmittel zur Durchführung der 3. Stufe des erfindungsgemäßen Verfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether wie Diethylether, Dioxan, Tetrahydrofuran oder Ethylenglykoldimethyl- oder -diethylether, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die 3. Stufe des erfindungsgemäßen Verfahrens wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide oder -alkoholate, wie beispielsweise Natriumhydrid, Natriumamid, Natriumhydroxid, Natriummethylat, Natriumethylat oder Kalium-t-butylat.

Die Reaktionstemperaturen können bei der Durchführung der 3. Stufe des erfindungsgemäßen Verfahrens in einem größeren Bereich variiert werden. Im allgemeinen arbeitet man bei Temperaturen von -70° C bis 100° C, vorzugsweise bei Temperaturen von -30° C bis 50° C.

Zur Durchführung der 3. Stufe des erfindungsgemäßen Verfahrens setzt man pro Mol an Alkoxymethylencarbonester der Formel (V) im allgemeinen 1.0 bis 1.5 Mol, vorzugsweise 1.0 bis 1.2 Mol an Keton oder Aldehyd der Formel (VI) und gegebenenfalls 1.0 bis 3.0 Mol, vorzugsweise 1.0 bis 1.5 Mol an Base ein.

Vorzugsweise mischt man zuerst die Reaktionspartner Alkoxymethylencarbonester der Formel (V) und Keton oder Aldehyd der Formel (VI) in einem geeigneten Verdünnungsmittel und erzeugt dann durch Zugabe der Base das reagierende Phosphonatanion in Anwesenheit der Carbonylverbindung in situ.

Die Reaktionsdurchführung, Aufarbeitung und Isolierung der Reaktionsprodukte erfolgt nach bekannten Methoden (vergleiche z. B. J. Amer. Chem. Soc. 83, 1734 [1961]).

Die so erhältlichen Verbindungen der Formel (I) leiten sich vom Naturstoff Strobilurin A ab (vergleiche z. B. Liebigs Ann. Chem. 1984, 1616 - 1625).

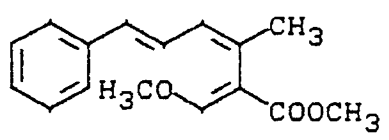

Strobilurin A

($R^1$=H; $R^2$=$CH_3$; $R^3$=$C_6H_5$; $R^4$=H)

Grundsätzlich können die Verbindungen (I) in 8 stereoisomeren Formen vorkommen, da sie 3 konjugierte Doppelbindungen enthalten. Die Stereochemie der Verbindungen (I) ist abhängig von der Stereochemie der Ausgangsverbindungen (V), wobei jedoch durch Isomerisierungen der Anionen der Verbindungen (V) im Verlauf der 3. Stufe des erfindungsgemäßen Verfahrens, sich die Stereoisomerenverteilung auch verschieben kann.

Da jedoch bei den Ausgangsverbindungen (V) das E,E-Isomere das stabilste ist und im Ausgangsprodukt dominiert und die 3. Stufe des erfindungsgemäßen Verfahrens mit einer hohen E-Selektivität abläuft überwiegt auch im Endprodukt der Formel (I) der Anteil des E,E,E-Isomeren.

Seine Abtrennung von den übrigen Isomeren ist durch Kristallisation beispielsweise aus Methanol möglich. Die photochemische Isomerisierung des E,E,E-Isomeren, zum E,Z,E-Isomeren, welches in der Stereochemie dem Naturstoff Strobilurin A entspricht, ist literaturbekannt (vergleiche z. B. DE-OS 30 25 368).

Die Verbindungen der Formel (I) sind entweder bekannt (vergleiche z. B. Liebigs Ann. Chem. 1984, 1616 - 1625; J. Antibiot. 36, 661 - 666 [1983]; Collect. Czech. Chem. Commun. 48, 1508 - 1512, [1983]) oder Gegenstand eigener bisher unveröffentlichter Anmeldungen (vergleiche DE 39 28 999 vom 01.09.1989) und lassen sich als Antibiotica, Fungizide oder Insektizide einsetzen.

Herstellungsbeispiele:

Beispiel 1:

1. Stufe:

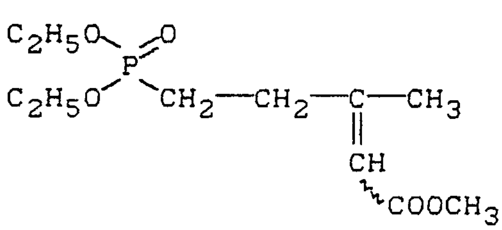

(IV-1)

Zu 12.2 g (0.109 Mol) Kalium-t-butylat in 100 ml Tetrahydrofuran gibt man bei einer Temperatur unter 20° C tropfenweise unter Rühren 19.8 g (0.108 Mol) Methoxycarbonylmethanphosphonsäuredimethylester und anschließend 21.6 g (0.104 Mol) γ-Ketobutylphosphonsäurediethylester (vergleiche J. Amer. Chem. Soc. 77, 3101 [1955]), rührt anschließend 12 Stunden bei Raumtemperatur, dekantiert dann die organische

Phase von abgeschiedenen Salz ab, engt im Vakuum ein, verteilt den Rückstand zwischen Essigester und Wasser, verfährt ebenso mit der abgeschiedenen Salzphase, engt die vereinigten Essigesterphasen im Vakuum ein und destilliert den Rückstand im Hochvakuum.

Man erhält 20.1 g (73.1 % der Theorie) an 4-(Diethoxyphosphinyl)-2-methyl-1-buten-1-carbonsäuremethylester vom Siedepunkt 130° C bis 140° C bei 1 Torr als Z/E-Isomerengemisch.

2. Stufe:

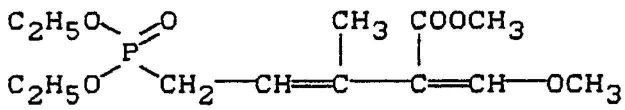

(V-1)

Zu einer Suspension von 4.5 g (0.15 Mol) 80prozentigem Natriumhydrid (in Mineralöl) in 75 ml Dimethylformamid gibt man bei Raumtemperatur tropfenweise unter Rühren 19.7 g (0.075 Mol) 4-(Diethoxyphosphinyl)-2-methyl-1-buten-1-carbonsäuremethylester in 44.8 g (0.75 Mol) Ameisensäuremethylester. Nach beendeter Gasentwicklung (ca. 30 Minuten) kühlt man die Reaktionsmischung auf 0° C ab und gibt nacheinander 7.2 g (0.075 Mol) Methansulfonsäure, 20.6 g (0.15 Mol) Kaliumcarbonat und 9.9 g (0.078 Mol) Dimethylsulfat zu, rührt dann 12 Stunden zunächst bei 0° C und später bei Raumtemperatur.
Zur Aufarbeitung gießt man die Reaktionsmischung in 500 ml Wasser und extrahiert mit Essigester. Die Essigesterphase wird eingeengt und destilliert.

Man erhält 17.9 g (78 % der Theorie) an 5-(Diethoxyphosphinyl)-1-methoxy-3-methyl-1,3-pentadien-2-carbonsäuremethylester als Isomerengemisch vom Siedebereich 140° C bis 160° C bei 0.5 Torr.
Im Rohprodukt sind außerdem noch Anteile zweier Nebenprodukte, welche im Phosphonesterteil basenkatalysierte Umesterungen erfahren haben. Es handelt sich hierbei um die Verbindungen 5-(Dimethoxyphosphinyl)-1-methoxy-3-methyl-1,3-pentadien-2-carbonsäuremethylester und 5-(Ethoxy-methoxyphosphinyl)-1-methoxy-3-methyl-1,3-pentadien-2-carbonsäuremethylester.
Die Nebenprodukte lassen sich massenspektometrisch charakterisieren.
Ihre Abtrennung ist nicht erforderlich, da sie sich in der folgenden 3. Stufe des erfindungsgemäßen Verfahrens in völlig gleicher Weise wie die oben beschriebene Diethoxyphosphinylmethylverbindung umsetzten.

3. Stufe:

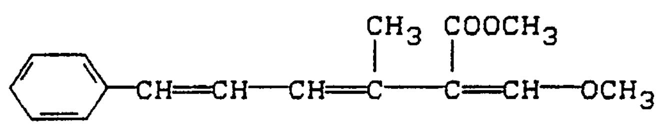

(I-1)

6.13 g (0.02 Mol) 5-(Diethoxyphosphinyl)-1-methoxy-3-methyl-1,3-pentadien-2-carbonsäuremethylester-Isomerengemisch und 2.12 g (0.02 Mol) Benzaldehyd in 20 ml Tetrahydrofuran werden bei -20° C unter kräftigem Rühren tropfenweise mit 2.25 g (0.02 Mol) Kalium-t-butylat in 20 ml Tetrahydrofuran versetzt, nach beendeter Zugabe auf Raumtemperatur gebracht, mit Ether verdünnt, mit Wasser gewaschen, über Natriumsulfat getrocknet und im Vakuum eingeengt.

Man erhält 4.85 g (94 % der Theorie) an rohem Isomerengemisch.
Durch Chromatographie in Cyclohexan : Essigester (5 : 1) oder durch Kristallisation aus Methanol erhält man hieraus 1.6 g (31 % der Theorie) an reinem E,E,E-1-Methoxy-3-methyl-6-phenyl-1,3,5-hexatrien-2-carbonsäuremethylester vom Schmelzpunkt 84° C bis 86° C.

Beispiel 2:

1. Stufe:

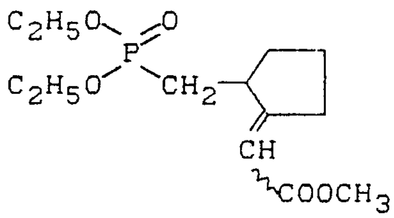

(IV-2)

Zu 5.6 g (0.05 Mol) Kalium-t-butylat in 50 ml Tetrahydrofuran gibt man bei 20° C tropfenweise unter Rühren 9.1 g (0.05 Mol) Methoxycarbonylmethanphosphonsäuredimethylester, dann 11.7 g (0.05 Mol) 2-Diethoxyphosphinylmethylcyclopentanon und rührt anschließend 12 Stunden bei Raumtemperatur. Zur Aufarbeitung dekantiert man die Tetrahydrofuranlösung von abgeschiedenen Salz ab, engt im Vakuum ein, verteilt den Rückstand zwischen Wasser und Essigester, verfährt ebenso mit der abgeschiedenen Salzphase, engt die vereinigten Essigesterphasen im Vakuum ein und destilliert den Rückstand im Hochvakuum.

Man erhält 11.8 g (81 % der Theorie) an 1-Methoxycarbonylmethyliden-2-(diethoxyphosphinylmethyl)-cyclopentan vom Siedebereich 132° C bis 142° C bei 0,2 Torr als E/Z-Isomerengemisch.

2. Stufe:

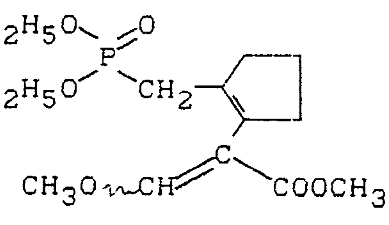

Zu einer Suspension von 6 g (0.2 Mol) 80prozentigem Natriumhydrid (in Mineralöl) in 100 ml Dimethylformamid gibt man bei Raumtemperatur tropfenweise unter Rühren 29 g (0.1 Mol) 1-Methoxycarbonylmethyliden-2-(diethoxyphosphinylmethyl)-cyclopentan in 60 g (1 Mol) Ameisensäuremethylester gelöst zu. Nach beendeter Gasentwicklung (ca. 30 Minuten) kühlt man die Reaktionsmischung auf 0° C ab und gibt nacheinander 9.6 g (0.1 Mol) Methansulfonsäure, 27.6 g (0.2 Mol) Kaliumcarbonat und 13.2 g (0.105 Mol) Dimethylsulfat zu und rührt dann 12 Stunden zunächst bei 0° C und später bei Raumtemperatur.

Zur Aufarbeitung gießt man die Reaktionsmischung in 500 ml Wasser und extrahiert mit Essigester. Die Essigesterphase wird eingeengt und destilliert.

Man erhält 28.3 g (85 % der Theorie) an 2-[(2-Diethoxyphosphinylmethyl)-cyclopenten-1-yl]-3-methoxyacrylsäuremethylester als Isomerengemisch vom Siedebereich 156° C bis 164° C bei 0.2 Torr.

Im Rohprodukt sind außerdem noch Anteile im Phosphonesterteil umgeesterter Nebenprodukte wie beispielsweise 2-[(2-Dimethoxyphosphinylmethyl)-cyclopenten-1-yl]-3-methoxyacrylsäuremethylester und 2-[(2-Methoxy-ethoxyphosphinylmethyl)-cyclopenten-1-yl]-3-methoxyacrylsäuremethylester enthalten, die sich jedoch in der folgenden 3. Stufe in gleicher Weise wie die oben beschriebene Diethoxyphosphinylmethyl-Verbindung umsetzen.

2. Stufe (Alternative):

20,3 g (0,07 Mol) 1-Methoxycarbonylmethyliden-2-(diethoxyphosphinylmethyl)-cyclopentan werden in 42 g (0,7 Mol) Ameisensäuremethylester und 140 ml Dimethylformamid gelöst. Man streut bei Raumtemperatur im Verlauf einer Stunde in 10 Portionen insgesamt 7,6 g (0,14 Mol) Natriummethylat ins Reaktionsgemisch ein. Man kühlt die Reaktionsmischung auf 0° C ab und gibt nacheinander 6,7 g (0,07 Mol) Methansulfonsäure, 19,3 g (0,14 Mol) Kaliumcarbonat und 8,9 g (0,07 Mol) Dimethylsulfat zu und rührt dann 12 Stunden zunächst bei 0° C und später bei Raumtemperatur.

Zur Aufarbeitung gießt man die Reaktionsmischung auf 350 ml Wasser und extrahiert mit Essigester. Die Essigesterphase wird eingeengt und destilliert.

Man erhält 20,5 g (88,1 % der Theorie) an 2-[(2-Diethoxyphosphinylmethyl)-cyclopenten-1-yl]-3-methoxyacrylsäuremethylester als Isomerengemisch vom Siedebereich 156° C bis 164° C bei 0,2 Torr.

3. Stufe:

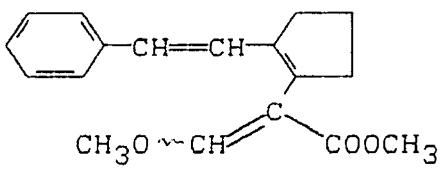

(I-2)

Zu 3.3 g (0.01 Mol) 2-[(2-Diethoxyphosphinylmethyl)-cyclopenten-1-yl]-3-methoxyacrylsäuremethylester und 1.05 g (0.01 Mol) Benzaldehyd in 10 ml Tetrahydrofuran gibt man bei -20° C tropfenweise unter kräftigem Rühren 1.1 g (0.01 Mol) Kalium-t-butylat in 10 ml Tetrahydrofuran zu, läßt das Gemisch nach beendeter Zugabe auf Raumtemperatur kommen, verdünnt mit Ether, wäscht mit Wasser, trocknet über Natriumsulfat und engt im Vakuum ein.

Aus dem Rohprodukt erhält man durch Umkristallisieren in Methanol und Toluol 1.4 g (50 % der Theorie) an 2-[(2-Phenylethenyl)-cyclopenten-1-yl]-3-methoxyacrylsäuremethylester vom Schmelzpunkt 123° C - 125° C.

Herstellung der Ausgangsverbindung:

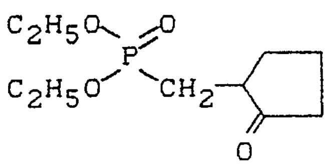

(II)

18.7 g (0.066 Mol) 2-Dimethylaminomethylcyclopentanonmethjodid (vergleiche J. Chem. Soc. 1958, 343) werden mit 55 g (0.33 Mol) Phosphorigsäuretriethylester 2 Stunden auf 150° C erhitzt; zur Aufarbeitung filtriert man ausgefallenes Trimethylethylammoniumiodid ab, engt im Vakuum ein und destilliert den Rückstand im Hochvakuum.

Man erhält 12.5 g (81 % der Theorie) an 2-(Diethoxyphosphinylmethyl)-cyclopentanon vom Siedepunkt 130° C bei 1 Torr.

Beispiel 3:

Völlig analog zu Beispiel 2 erhält man

1. Stufe:

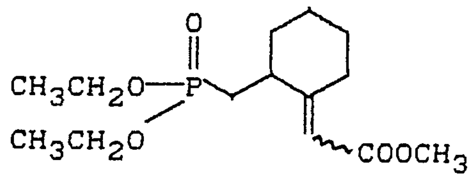

Siedebereich
138-140° C/0,1 Torr

2. Stufe:

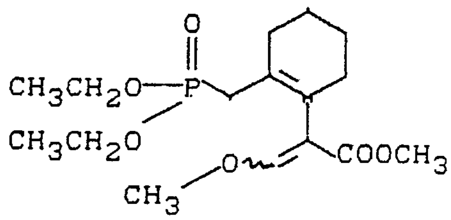

Siedebereich
165-173° C/0,2 Torr

3. Stufe:

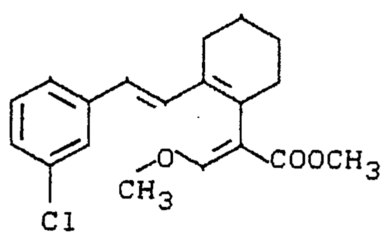

Schmelzpunkt
86-89° C

## Patentansprüche

**1.** Verfahren zur Herstellung von 1-Alkoxyhexatrien-2-carbonsäureestern der allgemeinen Formel (I),

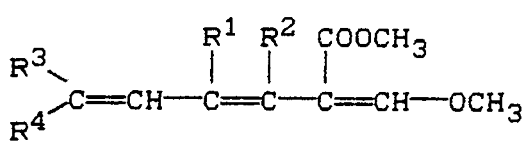

(I)

in welcher

$R^1$ für Wasserstoff, für gegebenenfalls substituiertes Alkyl, für Alkenyl, Alkinyl, für Cycloalkyl, Cycloalkenyl, für Phenyl oder für Heterocyclyl steht oder für einen Rest der Formel $-O-R^5$; $-S-R^5$ oder

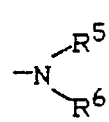

steht,

$R^2$ für Wasserstoff, für gegebenenfalls substituiertes Alkyl, für Alkenyl, Alkinyl, für Cycloalkyl, Cycloalkenyl, für Phenyl oder für Heterocyclyl steht oder

$R^1$ und $R^2$ gemeinsam mit den Kohlenstoffatomen, an sie welche ebunden sind, für einen jeweils gegebenenfalls substituierten Carbo- oder Heterocyclus stehen und

$R^3$, $R^4$, $R^5$ und $R^6$ unabhängig voneinander jeweils für Wasserstoff, für gegebenenfalls substituiertes Alkyl, für Alkenyl, Alkinyl, für Cycloalkyl, Cycloalkenyl, für gegebenenfalls substituiertes Phenyl oder für gegebenenfalls substituiertes Heterocyclyl stehen, wobei

$R^3$ und $R^4$ auch gemeinsam für einen zweifach verknüpften Alkandiylrest stehen können,

dadurch gekennzeichnet, daß man Oxophosphonsäureester der Formel (II),

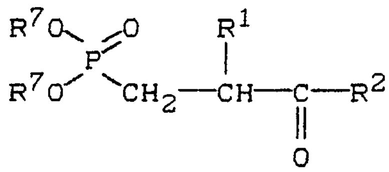

(II)

in welcher

$R^1$ und $R^2$ die oben angegebene Bedeutung haben und

$R^7$ für Wasserstoff, für gegebenenfalls substituiertes Alkyl, für Alkenyl, Alkinyl, für Cycloalkyl, Cycloalkenyl, für Phenyl oder für Heterocyclyl steht,

zunächst in einer ersten Stufe mit Alkoxycarbonylalkylphosphonester-Derivaten der Formel (III),

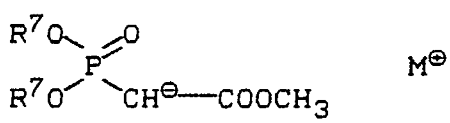

$M^{\oplus}$ (III)

in welcher

$R^7$ die oben angegebene Bedeutung hat und

$M^{\oplus}$ für ein Alkalimetallkation steht,

in Gegenwart eines Verdünnungsmittels und gegebenenfalls in Gegenwart einer Base umsetzt, anschließend in einer zweiten Stufe die so erhältlichen Acrylesterderivate der Formel (IV),

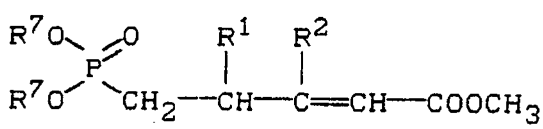

(IV)

in welcher

$R^1$, $R^2$ und $R^7$ die oben angegebene Bedeutung haben,

mit Ameisensäuremethylester in Gegenwart eines Verdünnungsmittels und in Gegenwart einer Base formyliert, dann entweder direkt in Anschluß daran in einem Reaktionsschritt oder auch in einer getrennten Reaktionsstufe mit einem Methylierungsmittel gegebenenfalls in Gegenwart eines Verdünnungsmittels und gegebenenfalls in Gegenwart einer Base methyliert und in einer letzten Stufe die so erhältlichen Alkoxymethylencarbonester der Formel (V),

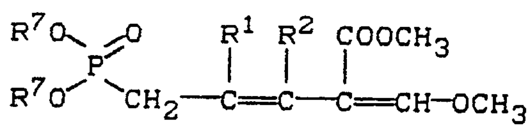

(V)

in welcher

$R^1$, $R^2$ und $R^7$ die oben angegebene Bedeutung haben,

mit Ketonen oder Aldehyden der Formel (VI),

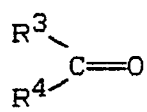

(VI)

in welcher

$R^3$ und $R^4$ die oben angegebene Bedeutung haben,

gegebenenfalls in Gegenwart eines Verdünnungsmittels und in Gegenwart einer Base umsetzt.

**2.** Verfahren gemäß Anspruch 1, dadurch gekennzeichnet, daß man Verbindungen der Formel (I), bei welchen

$R^1$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen: Halogen, Cyano, Nitro, jeweils geradkettiges oder verzweigtes Alkenyl, Alkinyl, Alkoxy, Alkenyloxy, Alkinyloxy, Alkylthio, Alkylendioxy, Alkoxycarbonyl oder Alkoximino mit jeweils 1 bis 6 Kohlenstoffatomen, jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, Cycloalkyl oder Cycloalkenyl mit jeweils 3 bis 7 Kohlenstoffatomen oder jeweils gegebenenfalls ein- bis fünffach gleich oder verschieden substituiertes Phenyl, Phenoxy, Phenylthio, Benzyloxy oder Benzylthio, wobei als Phenylsubstituenten in den letztgenannten Fällen jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^1$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht; und außerdem für einen Rest der Formel $-O-R^5$; $-S-R^5$ oder

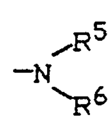

steht,

$R^2$ für Wasserstoff oder für gegebenenfalls einfach oder mehrfach, gleich oder verschieden substituiertes, geradkettiges oder verzweigtes Alkyl mit 1 bis 8

Kohlenstoffatomen steht, wobei als Substituenten die bei $R^1$ genannten Alkylsubstituenten infrage kommen; außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^1$ und $R^2$ gemeinsam mit den Kohlenstoffatomen, an welche sie gebunden sind, für einen jeweils gegebenenfalls ein- bis fünffach, gleich oder verschieden substituierten, fünf- bis siebengliedrigen Carbo- oder Heterocyclus mit 1 bis 3 Heteroatomen stehen, wobei als Heteroatome Stickstoff, Sauerstoff oder Schwefel infrage kommen und wobei als Substituenten jeweils infrage kommen: Halogen sowie jeweils geradkettiges oder verzweigtes Alkyl, Alkoxy oder Alkylthio mit jeweils 1 bis 4 Kohlenstoffatomen,

$R^3$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^1$ genannten Alkylsubstituenten infrage kommen; außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen oder für jeweils gegebenenfalls einfach oder mehrfach gleich oder verschieden substituiertes Phenyl oder Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, und wobei als Substituenten jeweils infrage kommen: Halogen, jeweils geradkettiges oder verzweigtes Alkyl, Alkenyl, Alkinyl, Alkoxy, Alkenyloxy oder Alkinyloxy mit jeweils bis zu 4 Kohlenstofftomen, sowie jeweils gegebenenfalls ein- bis fünffach, gleich oder verschieden durch Halogen sowie jeweils geradkettiges oder verzweigtes Alkyl oder Alkoxy substituiertes Phenyl, Phenoxy oder Benzyloxy und

$R^4$, $R^5$ und $R^6$ unabhängig voneinander für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^1$ genannten Alkylsubstituenten infrage kommen; außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^3$ und $R^4$ gemeinsam für einen zweifach verknüpften Alkandiylrest mit 3 bis 6 Kohlenstoffatomen stehen, herstellt.

**3.** Verfahren gemäß Anspruch 1, dadurch gekennzeichnet, daß man Verbindungen der Formel (I), bei welchen

$R^1$ für Wasserstoff, Methyl, Ethyl, n- oder i-Propyl, n-, i-, s- oder t-Butyl, Methoxy, Ethoxy, n- oder i-Propoxy, Methylthio, Allyl, Propargyl, für Cyclohexyl, Cyclohexenyl, Cyclopentyl, Cyclopentenyl, für Phenyl oder für jeweils gegebenenfalls ein- bis fünffach gleich oder verschieden im Phenylteil substituiertes Benzyl oder Phenoxy steht, wobei als Substituenten jeweils infrage kommen: Fluor, Chlor, Brom, Methyl, Ethyl, n- oder i-Propyl, n-, i-, s- oder t-Butyl, Methoxy, Ethoxy, n-oder i-Propoxy, Methylthio, Ethylthio, Trifluormethyl, Trifluormethoxy, Difluormethoxy, Trifluormethylthio,

$R^2$ für Wasserstoff, Methyl, Ethyl, n- oder i-Propyl, n-, i-, s- oder t-Butyl, für Cyclopentyl, Cyclopentenyl, Cyclohexyl, Cyclohexenyl oder Phenyl steht oder

$R^1$ und $R^2$ gemeinsam mit den Kohlenstoffatomen, an welche sie gebunden sind, für einen jeweils gegebenenfalls ein- bis dreifach, gleich oder verschieden substituierten fünf- bis siebengliedrigen Carbo- oder Heterocyclus mit 1 bis 2 Heteroatomen stehen, wobei als Heteroatome Stickstoff, Sauerstoff oder Schwefel infrage kommen und wobei als Substituenten jeweils infrage kommen: Fluor, Chlor, Brom, Methyl, Ethyl, n-

oder i-Propyl, n-, i-, s- oder t-Butyl, Methoxy, Ethoxy, n- oder i-Propoxy, Methylthio, Ethylthio,

$R^3$ für gegebenenfalls ein- bis dreifach gleich oder verschieden substituiertes Phenyl oder für gegebenenfalls ein- bis zweifach gleich oder verschieden substituiertes Heteroaryl steht, wobei Heteroaryl für einen aromatischen 5-Ring- oder 6-Ring-Heterocyclus mit 1 oder 2 gleichen oder verschiedenen Heteroatomen aus der Gruppe Stickstoff, Sauerstoff oder Schwefel steht und wobei als Substituenten jeweils infrage kommen:

Fluor, Chlor, Brom, Methyl, Ethyl, n- oder i-Propyl, n-, i-, s- oder t-Butyl, Allyl, Propargyl, Methoxy, Ethoxy, n- oder i-Propoxy, Allyloxy, Propargyloxy sowie jeweils gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, substituiertes Phenyl, Phenoxy oder Benzyloxy,

$R^4$ für Wasserstoff, Methyl, Ethyl, n- oder i-Propyl, n-, i-, s- oder t-Butyl steht oder

$R^3$ und $R^4$ gemeinsam für einen 1,3-Propandiyl, 1,4-Butandiyl, 1,5-Pentandiyl oder 1,6-Hexandiylrest stehen,

herstellt.

4. Verfahren gemäß Anspruch 1, dadurch gekennzeichnet, daß man Verbindungen der Formel (I), bei welchen

$R^1$ für Wasserstoff, Methyl, Methoxy, Phenyl oder Phenoxy steht,

$R^2$ für Wasserstoff oder Methyl steht oder

$R^1$ und $R^2$ gemeinsam mit den Kohlenstoffatomen, an welche sie gebunden sind, für einen 5-gliedrigen oder 6-gliedrigen Carbo- oder Heterocyclus mit einem Sauerstoff oder Schwefel als Heteroatom stehen, der gegebenenfalls ein- bis dreifach durch Methyl substituiert sein kann,

$R^3$ für gegebenenfalls einfach oder zweifach, gleich oder verschieden substituiertes Phenyl oder für gegebenenfalls einfach oder zweifach, gleich oder verschieden substituiertes Heteroaryl steht, wobei als Heteroarylreste die folgenden infrage kommen:

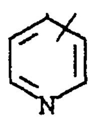 ; 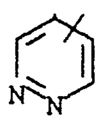 ; 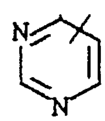 ; 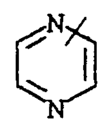 ;

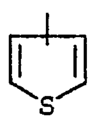 ; 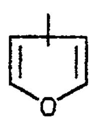 ; 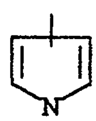 ; 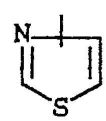 ;

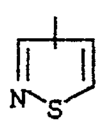 ; 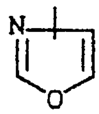 ; 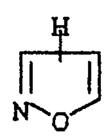

und wobei als Phenyl- oder Heteroarylsubstituenten jeweils infrage kommen: Fluor, Chlor, Brom, Methyl, Ethyl, n-oder i-Propyl, n-, i-, s- oder t-Butyl, Methoxy, Ethoxy, Allyloxy, Propargyloxy sowie jeweils gegebenenfalls ein- bis dreifach, gleich oder verschieden durch Fluor, Chlor, Brom, Methyl, Ethyl, Methoxy oder Ethoxy substituiertes Phenyl, Phenoxy oder Benzyloxy,

$R^4$ für Wasserstoff steht oder

$R^3$ und $R^4$ gemeinsam für einen 1,4-Butandiylrest oder einen 1,5-Pentandiylrest stehen,

herstellt.

5. Verfahren gemäß Anspruch 1, dadurch gekennzeichnet, daß man Verbindungen der Formel (I), bei welchen

$R^1$ für Wasserstoff und

$R^2$ für Methyl steht oder

$R^1$ und $R^2$ gemeinsam mit den Kohlenstoffatomen, an welche sie gebunden sind, für einen Rest der Formel

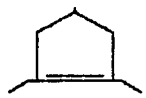 ; 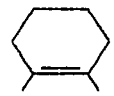 oder 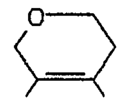

stehen,

$R^3$ für gegebenenfalls einfach oder zweifach, gleich oder verschieden substituiertes Phenyl steht, wobei als Substituenten infrage kommen: Chlor, Phenyl, Phenoxy oder Benzyloxy und wobei als Substitutionsstelle im Phenylring die 3-Position besonders bevorzugt ist und

$R^4$ für Wasserstoff steht,

herstellt.

**6.** Verfahren gemäß Anspruch 1, dadurch gekennzeichnet, daß man als Methylierungsmittel Dimethylsulfat, p-Toluolsulfonsäuremethylester, Methyliodid, Methylbromid oder Methylchlorid einsetzt.

**7.** Verfahren gemäß Anspruch 1, dadurch gekennzeichnet, daß man als Lösungsmittel aliphatische, alicyclische oder aromatische Kohlenwasserstoffe einsetzt.

**8.** Verfahren gemäß Anspruch 1, dadurch gekennzeichnet, daß man als Base Alkalimetallhydride, -hyroxide, -amide, -alkoholate, -carbonate oder -hydrogencarbonate einsetzt.

**9.** Acrylesterderivate der Formel (IVa),

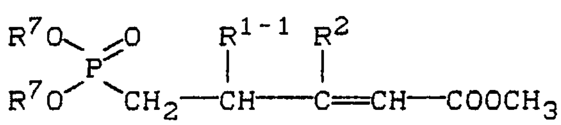

(IVa)

in welcher

$R^{1-1}$ für Wasserstoff, für gegebenenfalls substituiertes Alkyl, für Alkenyl, Alkinyl, für Cycloalkyl, Cycloalkenyl, für Phenyl oder für Heterocyclyl steht oder für einen Rest der Formel -O-$R^5$ oder -S-$R^5$ steht,

$R^2$ für Wasserstoff, für gegebenenfalls substituiertes Alkyl, für Alkenyl, Alkinyl, für Cycloalkyl, Cycloalkenyl, für Phenyl oder für Heterocyclyl steht oder

$R^{1-1}$ und $R^2$ gemeinsam mit den Kohlenstoffatomen, an welche sie gebunden sind, für einen jeweils gegebenenfalls substituierten Carbo- oder Heterocyclus stehen und

$R^5$ für Wasserstoff, für gegebenenfalls substituiertes Alkyl, für Alkenyl, Alkinyl, für Cycloalkyl, Cycloalkenyl, für gegebenenfalls substituiertes Phenyl oder für gegebenenfalls substituiertes Heterocyclyl steht, und

$R^7$ für Wasserstoff, für gegebenenfalls substituiertes Alkyl, für Alkenyl, Alkinyl, für Cyclkoalkyl, Cycloalkenyl, für Phenyl oder für Heterocyclyl steht.

**10.** Acrylesterderivate der Formel (IVa) gemäß Anspruch 9, in welcher

$R^{1-1}$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen: Halogen, Cyano, Nitro, jeweils geradkettiges oder verzweigtes Alkenyl, Alkinyl, Alkoxy, Alkenyloxy, Alkinyloxy, Alkylthio, Alkylendioxy, Alkoxycarbonyl oder Alkoximino mit jeweils 1 bis 6 Kohlenstoffatomen, jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, Cycloalkyl oder Cycloalkenyl mit jeweils 3 bis 7 Kohlenstoffatomen oder jeweils gegebenenfalls ein- bis fünffach gleich oder verschieden substituiertes Phenyl, Phenoxy, Phenylthio, Benzyloxy oder Benzylthio, wobei als Phenylsubstituenten in den letztgenannten Fällen jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^{1-1}$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht; und außerdem für einen Rest der Formel $-O-R^5$ oder $-S-R^5$ steht,

$R^2$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen; außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, oder

$R^{1-1}$ und $R^2$ gemeinsam mit den Kohlenstoffatomen, an welche sie gebunden sind, für einen jeweils gegebenenfalls ein- bis fünffach, gleich oder verschieden substituierten, fünf- bis siebengliedrigen Carbo-oder Heterocyclus mit 1 bis 3 Heteroatomen stehen, wobei als Heteroatome Stickstoff, Sauerstoff oder Schwefel infrage kommen und wobei als Substituenten jeweils infrage kommen: Halogen sowie jeweils geradkettiges oder verzweigtes Alkyl, Alkoxy oder Alkylthio mit jeweils 1 bis 4 Kohlenstoffatomen und

$R^5$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen;
außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht, und

$R^7$ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten die bei $R^{1-1}$ genannten Alkylsubstituenten infrage kommen;

$R^7$ außerdem für jeweils geradkettiges oder verzweigtes Alkenyl oder Alkinyl mit jeweils 2 bis 8 Kohlenstoffatomen, für Cycloalkyl oder Cycloalkenyl mit jeweils 3 bis 7 Kohlenstoffatomen, für Phenyl oder für Heterocyclyl steht, wobei Heterocyclyl für einen 5-Ring- oder 6-Ring-Heterocyclus mit 1 bis 3 gleichen oder verschiedenen Heteroatomen aus der Gruppe Stickstoff, Sauerstoff oder Schwefel steht.

**11.** Alkoxymethylencarbonester der Formel (V),

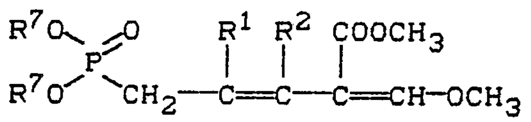

(V)

in welcher

$R^1$ für Wasserstoff, für gegebenenfalls substituiertes Alkyl, für Alkenyl, Alkinyl, für Cycloalkyl, Cycloalkenyl, für Phenyl oder für Heterocyclyl steht oder für einen Rest der Formel $-O-R^5$; $-S-R^5$ oder

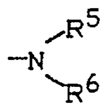

steht,

$R^2$ für Wasserstoff, für gegebenenfalls substituiertes Alkyl, für Alkenyl, Alkinyl, für Cycloalkyl, Cycloalkenyl, für Phenyl oder für Heterocyclyl steht oder

$R^7$ für Wasserstoff, für gegebenenfalls substituiertes Alkyl, für Alkenyl, Alkinyl, für Cycloalkyl, Cycloalkenyl, für Phenyl oder für Heterocyclyl steht.

**12.** Alkoxymethylencarbonate der Formel (VII),

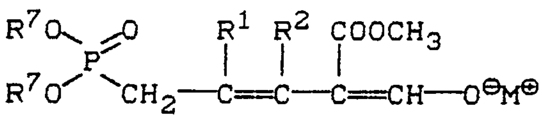

(VII)

in welcher

$R^1$, $R^2$ und $R^7$ die in Anspruch 11 angegebene Bedeutung haben und

$M^{\oplus}$ für Wasserstoff oder das Äquivalent eines Alkali- oder Erdalkalikation steht.

**13.** Verwendung der Verbindungen der Formel (V) und (VII) gemäß den Ansprüchen 11 und 12 als Zwischenprodukte und Ausgangsstoffe zur Herstellung von Verbindungen der Formel (I) gemäß Anspruch 1.